# EUROPEAN PATENT APPLICATION

(11) **EP 3 919 088 A1**
(43) Date of publication of application: **08.12.2021**
(21) Application number: 19913562.5
(22) Date of filing: 29.05.2019
(51) Int. Cl.: A61L 9/18, B01D 46/00

(54) **AIR STERILIZATION APPARATUS AND FILTER**

(30) Priority: 30.01.2019 CN 201910097743
(71) Applicant: Godox Photo Equipment Co., Ltd., Tangwei Community, Baoan District Shenzhen, Guangdong 518000 (CN)
(72) Inventor: ZENG, Weijun, Shenzhen, Guangdong 518000 (CN)
(74) Representative: Studio Torta S.p.A.
(86) International application number: PCT/CN2019/089109
(87) International publication number: WO 2020/155487

(57) **Abstract**

An air sterilization apparatus, comprising: a main housing (100). A cavity (101) for air circulation is provided in the main housing (100). The cavity (101) is provided with an air inlet (102) and an air outlet (103). The air inlet (102) and the air outlet (103) each is provided with a joint. A pulse flashing unit (300) is provided in the cavity (101). The pulse flashing unit (300) comprises a charging circuit (301) connected to an external power supply. A current output by the charging circuit (301) sequentially passes through a capacitor (302) and a discharging circuit (303), and is then input into an inert gas bulb (304). Strong light emitted by the pulse flashing unit (300) kills bacteria in air. No chemical residues would be produced, and the sterilization efficiency is high.

## Description

### TECHNICAL FIELD

The present invention relates to furniture and home appliances, and more particularly to an air sterilization device and an air filtration device.

### BACKGROUND

With the continuous improvement of people's living standards, people have put forward higher requirements for environmental health, and thus, air sterilization devices have also been widely adopted. The existing air sterilization devices mainly adopt chemical drugs or ultraviolet radiation to sterilize and disinfect, and they are usually adopted in places such as biopharmaceuticals, hospitals, airport terminals, and human settlements.

### SUMMARY

### TECHNICAL PROBLEM

In the existing air sterilization device, when the chemical medicine is used for sterilization, the chemical residue is likely to cause secondary pollution. In the ultraviolet radiation sterilization method, the personnel need to be evacuated during work. Moreover, the sterilization efficiency is low due to long-term exposure of ultraviolet rays, and the sterilization effect in a working environment with a fast air flow rate is not ideal.

### TECHNICAL SOLUTION

The purpose of the present invention is achieved through the following technical solutions.

The present invention provides an air sterilization device, comprising: a main housing; wherein a cavity for air circulation is provided in the main housing; the cavity is provided with an air inlet and an air outlet; each of the air inlet and the air outlet is provided with a joint; the cavity is provided with a pulse flashing unit; the pulse flashing unit comprises a charging circuit connected to an external power supply; a current output by the charging circuit passes through a capacitor and a discharging circuit in sequence, and then inputs to an inert gas lamp.

### BENEFICIAL EFFECTS

The air sterilization device provided in the present invention is equipped with a pulse flashing unit in the air flow path, such that bacteria in the air are killed through the strong light emitted by the pulse flashing unit. Therefore, the air sterilization device does not produce chemical residues, which also has a high sterilization efficiency and an ideal sterilization effect.

### BRIEF DESCRIPTION OF THE DRAWINGS

For ease of description, the present invention is described in detail with the following preferred embodiments and accompanying drawings.
FIG. 1 is an exploded structural diagram of an air sterilization device according to an embodiment of the present invention.
FIG. 2 is a cross-sectional structural diagram of the air sterilization device according to an embodiment of the present invention.
FIG. 3 is an overall structural diagram of the air sterilization device according to an embodiment of the present invention.

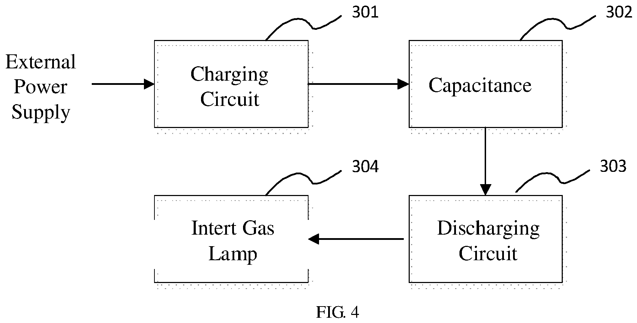

FIG. 4 is a schematic diagram of a circuit structure of a pulse flashing unit according to an embodiment of the present invention.

### DETAILED DESCRIPTION

### PREFERRED EMBODIMENT OF PRESENT INVENTION

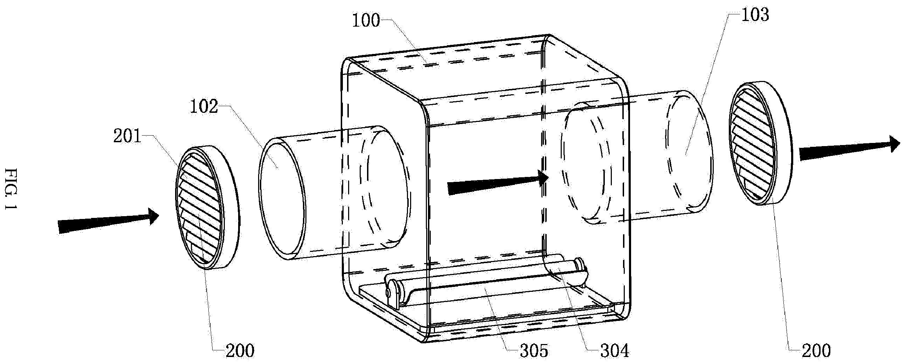

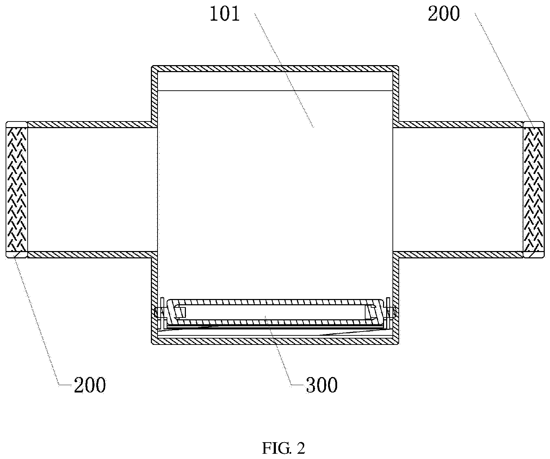

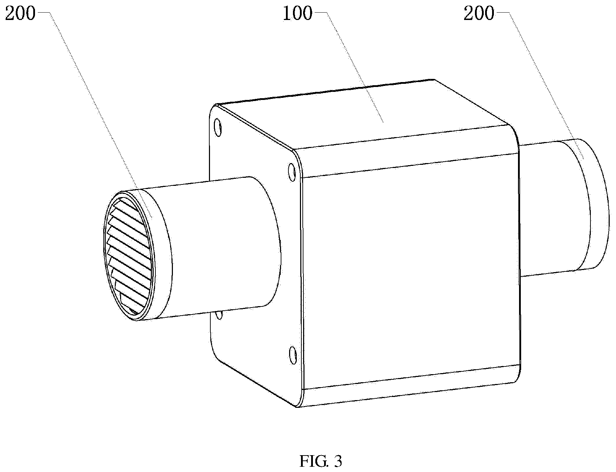

Referring to Figs. 1-3, the following embodiment specifically describes an air sterilization device, including: a main housing 100, and a cavity 101 for air circulation is provided in the main housing 100. The cavity 101 is provided with an air inlet 102 and an air outlet 103, and each of the air inlet 102 and the air outlet 103 is provided with a joint, which is provided for connecting to other external device, such as a fan. The cavity 101 is provided with a pulse flashing unit 300, and the pulse flashing unit 300 includes a charging circuit connected to an external power supply, and the current output by the charging circuit passes through a capacitor and a discharging circuit in sequence, and then inputs to the inert gas lamp. When the air sterilization device of present invention is working, the air enters the cavity 101 from the air inlet 102. The pulse flashing unit 300 in the cavity 101 generates strong flashing light to irradiate the air in the cavity 101, and effectively sterilizes the air passing through the cavity 101 through the pulsed strong light sterilization technology. And the irradiated air is discharged from the air outlet 103. The pulsed strong light sterilization technology is a world-leading light source technology, in which the pulse engineering technology for instantaneous discharging and a special inert gas lamp are adopted to stimulate strong white light in the form of pulses. The spectral distribution of the strong white light is similar to sunlight, and the light intensity thereof is equivalent to thousands or even tens of thousands of times the intensity of sunlight reaching the surface of the earth. In the pulsed strong light sterilization technology, instantaneous and high-intensity pulsed light energy is adopted to kill all kinds of microorganisms, thereby making up for the shortcomings of traditional heat sterilization and chemical sterilization. Moreover, due to the characteristics of short sterilization treatment time, the pulsed strong light sterilization technology is especially suitable for the working environment of air sterilization device with high air fluidity.

The working principle of the pulse flashing unit 300 is described as follows. The charging circuit 301 receives the current input from the external power supply and charges the capacitor 302. When the capacitor 302 is fully charged, the discharging circuit 303 drives the capacitor 302 to release a large current in an instant to drive the inert gas lamp 304 to emit a brief and intense white light.

Referring to Figs. 1-3, the following embodiment specifically describes an air sterilization device of the present invention, including: a main housing 100, and a cavity 101 for air circulation is provided in the main housing 100. The cavity 101 is provided with an air inlet 102 and an air outlet 103, and each of the air inlet 102 and the air outlet 103 is provided with a joint, which is provided for connecting to other external device, such as a fan. The cavity 101 is provided with a pulse flashing unit 300, and the pulse flashing unit 300 includes a charging circuit connected to an external power supply, and the current output by the charging circuit passes through a capacitor and a discharging circuit in sequence, and then inputs to the inert gas lamp. When the air sterilization device of present invention is working, the air enters the cavity 101 from the air inlet 102. The pulse flashing unit 300 in the cavity 101 generates strong flashing light to irradiate the air in the cavity 101, and effectively sterilizes the air passing through the cavity 101 through the pulsed strong light sterilization technology. And the irradiated air is discharged from the air outlet 103. The pulsed strong light sterilization technology is a world-leading light source technology, in which the pulse engineering technology for instantaneous discharging and a special inert gas lamp are adopted to stimulate strong white light in the form of pulses. The spectral distribution of the strong white light is similar to sunlight, and the light intensity thereof is equivalent to thousands or even tens of thousands of times the intensity of sunlight reaching the surface of the earth. In the pulsed strong light sterilization technology, instantaneous and high-intensity pulsed light energy is adopted to kill all kinds of microorganisms, thereby making up for the shortcomings of traditional heat sterilization and chemical sterilization. Moreover, due to the characteristics of short sterilization treatment time, the pulsed strong light sterilization technology is especially suitable for the working environment of air sterilization device with high air fluidity.

The working principle of the pulse flashing unit 300 is described as follows. The charging circuit 301 receives the current input from the external power supply and charges the capacitor 302. When the capacitor 302 is fully charged, the discharging circuit 303 drives the capacitor 302 to release a large current in an instant to drive the inert gas lamp 304 to emit a brief and intense white light.

In order to increase the air flow speed in the cavity 101, a fan can be connected to the joint of the air inlet 102 or the air outlet 103 of the cavity 101, so that external air can enter the cavity 101 from the air inlet 102, and then is discharged from the air outlet 103. In specific use, the positions of the air inlet 102 and the air outlet 103 can be exchanged according to actual requirements.

In the embodiment, each of the air inlet 102 and the air outlet 103 of the cavity 101 is covered with a light-blocking assembly 200, and the light-blocking assembly 200 includes at least two light barriers, and a space for air to pass is provided between two adjacent light barriers. The light barriers are inclined to a light emitting surface of the inert gas lamp. Two light-blocking assemblies 200 are arranged between the filter assembly and the fan, to effectively prevent the strong light from leaking out of the main housing 100 and causing damage to the user's eyes. A louvered structure is adopted between two adjacent light barriers, which can effectively ensure that the air can smoothly pass through the cavity 101 while preventing the leakage of strong light.

In the embodiment, the number of the pulse flashing unit 300 is at least one, and the pulse flashing unit 300 is fixedly arranged on the inner side wall of the cavity 101. Specifically, the number of the pulse flashing units 300 can be increased or decreased according to actual conditions. When the air circulation speed is relatively fast, the number of the pulse flashing units 300 can be set to two, and each pulse flashing unit 300 flashes in sequence, thereby effectively shortening the flash cycle.

In the embodiment, a lamp shade 305 is provided on the outside of the inert gas lamp, and the lamp shade 305 is used to adjust the direction of light emission, thereby effectively improving the working efficiency of the product.

In the embodiment, the surface of the light barrier is coated with a light-absorbing material. Specifically, the light-absorbing material refers to a material that absorbs light and then diffusely reflects part of the light. When light shines on the light-absorbing material, there is no transmission outside of the illumination, nor does it produce mapping, large flares and reflections.

In the embodiment, the inert gas lamp is a xenon lamp. The xenon lamp refers to a bulb filled with an inert gas mixture including xenon.

In an embodiment, provided is an air sterilization and filtration device, including the air sterilization device as described above, and the joint on the air inlet 102 or the air outlet 103 of the air sterilization device is connected to a fan. The air outlet surface of the fan may be connected to the interface of the air inlet 102, so that the external air enters the cavity 101 after passing through the fan, and then is discharged from the air outlet 103 of the cavity 101. Or, the air inlet surface of the fan is connected to the interface of the air outlet 103, so that the external air enters the cavity 101 from the air inlet 102 of the cavity 101, and enters the air inlet surface of the fan from the air outlet 103 of the cavity 101, and then is discharged from the fan air outlet surface of the fan.

In the description of this specification, the description with reference to the terms "one embodiment", "some embodiments", "exemplary embodiments", "examples", "specific examples" or "some examples" means that the specific feature, structure, material or characteristic described in combination with the embodiment or example is included in at least one embodiment or example of the present invention. Moreover, the described specific features, structures, materials or characteristics can be combined in an appropriate manner in any one or more embodiments or examples.

Described above are only the preferred embodiments of the present invention and are not intended to limit the present invention. Any modification, equivalent replacement and improvement made within the spirit and principle of the present invention shall be included in the protection scope of the present invention.

### Industrial applicability

The air sterilization device of the present invention can be applied to various densely populated scenes, which has extremely high industrial applicability.

## Claims

1. An air sterilization device, comprising:
a main housing;
wherein a cavity for air circulation is provided in the main housing; the cavity is provided with an air inlet and an air outlet; each of the air inlet and the air outlet is provided with a joint; the cavity is provided with a pulse flashing unit; the pulse flashing unit comprises a charging circuit connected to an external power supply; a current output by the charging circuit passes through a capacitor and a discharging circuit in sequence, and then inputs to an inert gas lamp.

2. The air sterilization device of claim 1, wherein each of the air inlet and the air outlet of the cavity is covered with a light-blocking assembly; the light-blocking assembly comprises at least two light barriers; a space for air to pass is provided between two adjacent light barriers; and the light barriers are inclined to a light emitting surface of the inert gas lamp.

3. The air sterilization device of claim 2, wherein the number of the pulse flashing unit is at least one; and the pulse flashing unit is fixedly arranged on an inner side wall of the cavity.

4. The air sterilization device of claim 3, wherein an outer side of the inert gas lamp is provided with a lamp shade.

5. The air sterilization device of claim 4, wherein a surface of each of the light barriers is coated with light-absorbing material.

6. The air sterilization device of claim 5, wherein the inert gas lamp is a xenon lamp.

7. An air sterilization and filtration device, comprising the air sterilization device according to any one of claims 1 to 6, wherein a joint on an air inlet or an outlet of the air sterilization device is connected to a fan.
